# EUROPEAN PATENT APPLICATION

(11) **EP 4 461 717 A1**
(43) Date of publication of application: **13.11.2024**
(21) Application number: 23172988.0
(22) Date of filing: 12.05.2023
(51) Int. Cl.: C07C 1/24, C07C 2/76, C07C 15/04, C07C 15/06, C07C 15/08, C07C 11/04, B01J 29/40

(54) **TWO-STEP PROCESS FOR MANUFACTURING AROMATIC HYDROCARBONS FROM ALCOHOLS**

(71) Applicant: BASF SE, 67056 Ludwigshafen am Rhein (DE)
(72) Inventor: Jalapoor, Daniel, 69123 Heidelberg (DE); Roussiere, Thomas, 67056 Ludwigshafen am Rhein (DE); Braunsmann, Kirsten, 67056 Ludwigshafen (DE); Lejkowski, Michael Ludwig, 67056 Ludwigshafen am Rhein (DE); Czaja, Alexander, 69123 Heidelberg (DE); Kindler, Alois, 67056 Ludwigshafen am Rhein (DE); Collins, Lee Russell, 67056 Ludwigshafen am Rhein (DE)
(74) Representative: Altmann Stößel Dick Patentanwälte PartG mbB

(57) **Abstract**

The present invention relates to a process for converting alcohols to aromatic hydrocarbons comprising, (1) preparing a feed comprising one or more alcohols having two or more carbon atoms; (2) providing a dehydration catalyst; (3) contacting the feed prepared in (1) with the dehydration catalyst provided in (2) and conducting a reaction, obtaining a mixture comprising one or more olefin and water; (4) optionally separating at least a portion of the water from the mixture obtained in (3) or optionally adding water to the mixture obtained in (3); (5) providing a catalyst comprising a zeolite; (6) contacting the mixture obtained in (3) or in (4) with the catalyst provided in (5), obtaining a mixture comprising one or more aromatic hydrocarbons as a reaction product; and (7) separating at least a part of the aromatic hydrocarbons from the reaction product obtained in (6).

## Description

### TECHNICAL FIELD

The present invention relates to a process for the production of aromatic hydrocarbons by the catalytic conversion of a feed comprising one or more alcohols having two or more carbon atoms.

### INTRODUCTION

In many technical processes, the use of zeolites has gained great importance due to their properties as shape selective catalysts in connection with suitable acid properties. In the following, an overview of the state of the art is given in the field of various applications in which zeolite catalysts are being successfully used.

Uslamin et al., Chemical Engineering Science 198 (2019) 305-316, concerns gallium-promoted HZSM-5 zeolites that can be used as efficient catalysts for the aromatization of biomass-derived furans.

Zhang et al., Science 2020, 370, 437-441, relates to the upcycling of polyethylene to long-chain alkylaromatics by tandem hydrogenolysis/aromatization. The publication from Zhang et al. deals with the catalytic reactions which are associated with the upgrading of plastic materials from waste. One of the goals of the upcycling is the production of mixtures of benzene, toluene, and the three xylene isomers (BTX), whereby the use of metal loaded zeolites is shown for certain process routes. As metal loaded zeolites a Mo/ZSM-5 is mentioned for the conversion of methane at temperature in the range from 900 - 1 ,000°C and ZnZSM-5 or Ga/ZSM-5 for the aromatization of C2 to C6 hydrocarbons.

Uslamin et al., ACS Catal. 2019, 9, 8547-8554 concerns the co-aromatization of furan and methanol over ZSM-5. Uslamin et al. describes a process for the conversion of furan and methanol to bio-aromatics which is catalyzed by H-ZSM-5. The goal is to produce bio-aromatics in the presence of a ZSM-5 catalyst which does not contain a metal loading. The zeolite that was employed was an ammonium ZSM-5 zeolite with Si/AI ratio of 40. The ZSM-5 was calcined at 550 °C for 5 h to obtain the proton form HZSM-5.

US 9,533,921 B2 relates to the catalytic conversion of alcohols to hydrocarbons by using zeolites. US 9,533,921 B2 discloses the catalytic conversion of ethanol containing feeds in the presence of metal loaded ZSM-5 zeolite whereby the ethanol containing feeds can contain a high amount of water. US 9,533,921 B2 also mentions bimetallic metal loadings with two different metals (see column 6 of US 9,533,921 B2). Examples show bimetallic metals loadings with such as Cu and Fe on ZSM-5 (column 10 of US 9,533,921 B2).

Uslamin et al., Catal. Sci. Technol. 2020. 10, 2774, on the other hand, relates to the aromatization of ethylene using zeolite based catalysts, wherein in particular HZSM-5 which has been modified with Ga, Zn, and Ag leads to an increase in the production of BTX.

There however remains a need for an improved process for the manufacturing of benzene directly from ethanol, in particular in view of the availability of bio-ethanol as a sustainable source of carbon.

### DETAILED DESCRIPTION

Thus, it was the object of the present invention to provide an improved process for the production of aromatic hydrocarbons, preferably benzene, toluene and xylene, from a feed comprising ethanol, and in particular bio-ethanol.

Said object is achieved by the process of the present invention, consisting of a two-step catalyzed conversion of ethanol towards aromatic hydrocarbons. Surprisingly, it was found that the process of the present invention permits a highly efficient conversion of an ethanol comprising feed to aromatic hydrocarbons comprising benzene, toluene and xylene, in particular compared to a one step process starting either from ethanol or ethylene.

Therefore, the present invention relates to a process for converting alcohols to aromatic hydrocarbons comprising,
(1) preparing a feed comprising one or more alcohols having two or more carbon atoms;
(2) providing a dehydration catalyst;
(3) contacting the feed prepared in (1) with the dehydration catalyst provided in (2) and conducting a reaction, obtaining a mixture comprising one or more olefin and water;
(4) optionally separating at least a portion of the water from the mixture obtained in (3) or optionally adding water to the mixture obtained in (3);
(5) providing a catalyst comprising a zeolite;
(6) contacting the mixture obtained in (3) or in (4) with the catalyst provided in (5), obtaining a mixture comprising one or more aromatic hydrocarbons as a reaction product;
(7) separating at least a part of the aromatic hydrocarbons from the reaction product obtained in (6).

It is preferred that the one or more alcohols in (1) comprise, preferably consist of, one or more of ethanol, propanol, butanol, and isobutanol, preferably comprise, more preferably consist of, one or more of ethanol and propanol, more preferably comprise, more preferably consist of, ethanol.

It is preferred that the alcohol in (1) is the product of a fermentation process, preferably of the fermentation of biological material obtained from harvested plants and/or residual products from harvested plants.

It is preferred that the feed in (1) further comprises water. In case the feed in (1) comprises water, it is preferred that the water content of the feed in (1) is 80 vol.-% or higher, more preferably 85 vol.-% or higher, more preferably 90 vol.-% or higher, more preferably 95 vol.-% or higher.

It is preferred that, the dehydration catalyst in (2) comprises, preferably consists of, one or more acid catalysts, preferably one or more solid acid catalysts and/or ionic liquids, wherein the one or more solid acid catalysts preferably comprise, more preferably consist of, one or more selected from the group consisting of alumina, sulfated zirconia, and zeolites, including mixtures of two or more thereof, more preferably comprise, more preferably consist of, one or more selected from the group consisting of gamma-alumina and MFI-type zeolites, including mixtures of two or more thereof, more preferably comprise, more preferably consist of, one or more selected from the group consisting of activated gamma-alumina and ZSM-5, including mixtures of two or more thereof.

It is preferred that, the dehydration catalyst in (2) further comprises a binder, wherein the binder preferably comprises, more preferably consists of, one or more selected from the group consisting of titania, zirconia, alumina, silica, silica-alumina, titania-silica, titania-alumina, zirconia-silica, zirconia-alumina, titania-zirconia, and mixtures of two or more thereof, more preferably from the group consisting of silica-alumina, titania-silica, titania-alumina, zirconia-silica, zirconia-alumina, titania-zirconia, and mixtures of two or more thereof, wherein more preferably the binder comprises, more preferably consists of, silica-alumina.

It is preferred that the dehydration catalyst in (2) is provided as a shaped body, preferably as an extrudate.

It is preferred that from 95 to 100 wt.-% of the dehydration catalyst provided in (2) consists of the zeolite and the optional binder, preferably from 97 to 100 wt.-%, more preferably from 98 to 100 wt.-%, more preferably from 99 to 100 wt.-%, based on the total weight of the catalyst.

It is preferred that contacting in (3) is conducted at a temperature in the range of from 250 to 600 °C, preferably from 275 to 550 °C, more preferably 290 to 400 °C, more preferably from 300 to 450 °C, more preferably from 300 to 420 °C.

It is preferred that contacting in (3) is conducted at a pressure in the range of from 0.5 to 1.3 bara, preferably from 0.7 to 1.2 bara, more preferably from 0.9 to 1.1 bara.

It is preferred that the feed in (1) further comprises one or more inert gases, preferably nitrogen and/or argon, more preferably nitrogen.

It is preferred that the gas hourly space velocity at which the feed is contacted with the catalyst in (3) is in the range of from 600 to 2000 h⁻¹, preferably from 700 to 1600 h⁻¹, more preferably from 800 to 1300 h⁻¹, more preferably from 900 to 1050 h⁻¹.

It is preferred that the feed in (1) is a feed stream and contacting in (3) is conducted as a continuous process.

Alternatively, it is preferred that contacting in (3) is conducted as a batch process.

It is preferred that the mixture obtained in (3) comprises one or more of ethylene, propylene, and butene, preferably one or more of ethylene and propylene, more preferably ethylene.

It is preferred that the feed provided in (1) and contacted with the catalyst in (3) is in the liquid phase and/or in the gas phase, preferably in the gas phase.

Furthermore, it is preferred that the process further comprises
(8) recycling the remaining reaction product comprising one or more unreacted olefins obtained in (7) to the feed prepared in (1) and/or to the mixture obtained in (3) and/or to the mixture contacted with the catalyst in (6), preferably to the mixture obtained in (3) and/or to the mixture contacted with the catalyst in (6), more preferably to the mixture contacted with the catalyst in (6).

Alternatively, it is preferred that the process further comprises
(8) recycling one or more unreacted olefins obtained in (7) to the feed prepared in (1) and/or to the mixture obtained in (3) and/or to the mixture contacted with the catalyst in (6), preferably to the mixture obtained in (3) and/or to the mixture contacted with the catalyst in (6), more preferably to the mixture contacted with the catalyst in (6), wherein the one or more unreacted olefins comprise one or more olefins selected from the group consisting of C2 to C10 olefins, preferably C2 to C9 olefins, more preferably C2 to C8 olefins, more preferably C2 to C5 olefins, more preferably C2 to C4 olefins, more preferably C2 to C3 olefins, more preferably wherein the one or more unreacted olefins is ethylene.

It is preferred that the zeolite in (5) has an AEI, AFN, AFT, AFX, AFY, BSU, CGS, CHA, CON, DFT, EDI, ERI, ETV, IFY, IFW, IMF, ITH, ITR, IWR, JSR, JST, LOV, MFI, MER, MEL, OSO, PHI, POR, PUN, RSN, SAT, SBN, SFW, SIV, SOF, STW, SWY, SZR, THO, or UOS type framework structure, preferably an AEI, CGS, CHA, IFY, IMF, ITH, ITR, MFI, MER, POR, PUN, SZR, or UOS type framework structure, more preferably an MFI, MER, or SZR type framework structure, more preferably has an MFI type framework structure.

It is preferred that the zeolite in (5) has an MFI type framework structure, and wherein the zeolite is ZSM-5, Silicailite, Bor-C, LZ-105, AMS-1B, FZ-1, TZ-01, USC-4, NU-5, ZMQ-TB, TS-1, USI-108, AZ-1, TSZ, ZKQ-1B, NU-4, ZBH, or TSZ-III, preferably is ZSM-5.

It is preferred that the zeolite in (5) is a zeolite loaded with Ga, Zn, or Ni, or with combinations of two or more thereof, preferably with Zn or Ni, or with Zn and Ni, more preferably with Zn.

It is preferred that the zeolite in (5) is a zeolite loaded with Zn in the range of from 0.1 to 10 wt.-% calculated as the element and based on 100 wt.-% of the loaded zeolite, preferably from 0.2 to 8 wt.-%, more preferably from 0.3 to 7 wt.-%, more preferably from 0.5 to 5 wt.-%, more preferably from 0.7 to 3 wt.-%, more preferably from 0.9 to 1.5 wt.-%, more preferably from 1.0 to 1.3 wt.-%.

It is preferred that the zeolite in (5) is a zeolite loaded with Ga in the range of from 0.01 to 1 wt.-% calculated as the element and based on 100 wt.-% of the loaded zeolite, preferably from 0.05 to 0.6 wt.-%, more preferably from 0.075 to 0.5 wt.-%, more preferably from 0.1 to 0.4 wt.-%, more preferably from 0.15 to 0.3 wt.-%.

Alternatively it is preferred that the zeolite in (5) is a zeolite loaded with Ga in the range of from 1 to 5 wt.-% calculated as the element and based on 100 wt.-% of the loaded zeolite, preferably from 1.5 to 2.5 wt.-%, more preferably from 1.7 to 2.3 wt.-%, more preferably from 1.8 to 2.2 wt.-%, more preferably from 1.9 to 2.1 wt.-%.

It is preferred that the zeolite in (5) is a zeolite loaded with Ni in the range of from 0.1 to 3 wt.-% calculated as the element and based on 100 wt.-% of the loaded zeolite, preferably from 0.5 to 1.5 wt.-%, more preferably from 0.7 to 1.3 wt.-%, more preferably from 0.8 to 1.2 wt.-%, more preferably from 0.9 to 1.1 wt.-%.

It is preferred that the zeolite in (5) is further loaded with P, Mg, or Ca, or with combinations of two or more thereof, preferably with P or Mg, or with P and Mg, more preferably with P.

It is preferred that the zeolite in (5) is further loaded with P in the range of from 0.1 to 5 wt.-% calculated as the element and based on 100 wt.-% of the loaded zeolite, preferably from 1 to 2 wt.-%, more preferably from 1.2 to 1.8 wt.-%, more preferably from 1.3 to 1.7 wt.-%, more preferably from 1.4 to 1.6 wt.-%.

It is preferred that the zeolite in (5) is further loaded with Mg in the range of from 0.1 to 5 wt.-% calculated as the element and based on 100 wt.-% of the loaded zeolite, preferably from 1 to 2 wt.-%, more preferably from 1.2 to 1.8 wt.-%, more preferably from 1.3 to 1.7 wt.-%, more preferably from 1.4 to 1.6 wt.-%.

It is preferred that the zeolite in (5) is further loaded with Ca in the range of from 0.1 to 5 wt.-% calculated as the element and based on 100 wt.-% of the loaded zeolite, preferably from 1 to 2 wt.-%, more preferably from 1.2 to 1.8 wt.-%, more preferably from 1.3 to 1.7 wt.-%, more preferably from 1.4 to 1.6 wt.-%.

It is preferred that the zeolite in (5) comprises YO₂ and X₂O₃ in its framework structure, wherein Y stands for a tetravalent element and X stands for a trivalent element. In case the zeolite in (5) comprises YO₂ and X₂O₃ in its framework structure, X is preferably selected from the group consisting of Al, B, Ga, and combinations of two or more thereof, wherein X is more preferably Al. Further, it is preferred that Y is selected from the group consisting of Si, Ti, Sn, Ge, and combinations of two or more thereof, wherein Y is more preferably Si.

It is preferred that the zeolite in (5) comprises Si and Al in its framework structure, wherein the Si:Al molar ratio of the zeolite in (2) ranges from 1 to 150, preferably from 3 to 120, more preferably from 5 to 110, more preferably from 30 to 100, more preferably from 40 to 80, more preferably from 50 to 70.

It is preferred that the surface area of the zeolite in (5) ranges from 250 to 550 m²/g, preferably from 275 to 525 m²/g, more preferably from 300 to 500 m²/g, more preferably from 325 to 475 m²/g, more preferably from 350 to 450 m²/g.

It is preferred that the catalyst in (5) further comprises a binder, wherein the binder preferably comprises, more preferably consists of, one or more selected from the group consisting of titania, zirconia, alumina, silica, silica-alumina, titania-silica, titania-alumina, zirconia-silica, zirconia-alumina, titania-zirconia, and mixtures of two or more thereof, more preferably from the group consisting of silica-alumina, titania-silica, titania-alumina, zirconia-silica, zirconia-alumina, titania-zirconia, and mixtures of two or more thereof, wherein more preferably the binder comprises, more preferably consists of, silica-alumina.

It is preferred that the catalyst in (5) is provided as a shaped body, preferably as an extrudate.

It is preferred that from 95 to 100 wt.-% of the catalyst provided in (5) consists of the zeolite and the optional binder, preferably from 97 to 100 wt.-%, more preferably from 98 to 100 wt.-%, more preferably from 99 to 100 wt.-%, based on the total weight of the catalyst.

It is preferred that the process includes a step of regenerating the catalyst after contacting with the feed in (6) wherein the catalyst is preferably regenerated by calcination at a temperature in the range of from 300 to 800 °C, preferably from 350 to 700 °C, more preferably from 400 to 600 °C, more preferably from 450 to 500 °C. In case the process includes a step of regenerating the catalyst by calcination, it is preferred that the calcination is conducted for a period ranging from 1 to 24 h, more preferably from 2 to 12 h, more preferably from 3 to 8 h.

It is preferred that contacting in (3) or (6), preferably in (3) and (6), is conducted in a reactor, preferably in a fixed bed reactor or in a fluidized bed reactor, more preferably in a fixed bed reactor.

It is preferred that contacting in (6) is conducted at a temperature in the range of from 350 to 600 °C, preferably from 370 to 550 °C, more preferably 390 to 530 °C, more preferably from 410 to 500 °C, more preferably from 420 to 480 °C.

It is preferred that contacting in (6) is conducted at a pressure in the range of from 0.5 to 1.3 bara, preferably from 0.7 to 1.2 bara, more preferably from 0.9 to 1.1 bara.

It is preferred that the gas hourly space velocity at which the mixture obtained in (3) or (4) is contacted with the catalyst in (5) is in the range of from 1500 to 2500 h⁻¹, preferably from 1600 to 2200 h⁻¹, more preferably from 1700 to 2150 h⁻¹, more preferably from 1800 to 2100 h⁻¹, more preferably from 1900 to 2050 h⁻¹.

It is preferred that the mixture obtained in (3) or (4) is a feed stream and contacting in (6) is conducted as a continuous process, wherein preferably the process is conducted as a continuous process.

Alternatively, it is preferred that contacting in (6) is conducted as a batch process, wherein preferably the process is conducted as a batch process.

It is preferred that the aromatic hydrocarbons obtained in (6) comprise one or more of benzene, toluene, and xylene, preferably one or more of benzene and toluene, more preferably benzene.

It is preferred that the mixture obtained in (3) or (4) and contacted with the catalyst in (6) is in the liquid phase and/or in the gas phase, preferably in the gas phase.

It is preferred that the total yield of benzene, toluene, and xylene (BTX) obtained in (6) is 35 % or higher based on 100% of the one or more olefins which are converted in (6), preferably 40 % or higher, more preferably 45 % or higher, more preferably 50 % or higher, more preferably 55 % or higher.

It is preferred that (6) comprises at least one reaction stage (A), wherein in (A)
(6.1) contacting is carried out in at least two reactors RA1, ... , RAn connected in parallel, wherein each reactor contains the catalyst provided in (5);
(6.2) at a given point in time during contacting, at least one of the at least two reactors RA1, ... , RAn is out of operation for regenerating the catalyst present, so that at least 1 reactor remains in operation.

The term "connected in parallel" as used in this context relates to an apparatus set-up which comprises suitable means for distributing at least one feed stream to be introduced into stage (A) to the reactors RA1, .., RAn by dividing this feed stream into n substreams and by introducing each substream into an individual reactor.

In case (6) comprises at least one reaction stage (A), it is preferred that (6) further comprises (6.3) uniting the mixtures comprising one or more aromatic hydrocarbons as a reaction product obtained according to (6.1) of the at least two reactors RA1, ... , RAn.

It is preferred that the process is operated without using combustion as energy source, wherein the process is preferably operated using only electric energy as energy source, more preferably using only renewable electric energy as energy source, more preferably using only energy from renewable energy sources as energy source.

It is preferred that independently from each other separating in (7) is conducted by distillation, preferably by fractional distillation.

The present invention is further illustrated by the following set of embodiments and combinations of embodiments resulting from the dependencies and back-references as indicated. In particular, it is noted that in each instance where a range of embodiments is mentioned, for example in the context of a term such as "The process according to any one of embodiments 1 to 4", every embodiment in this range is meant to be explicitly disclosed for the skilled person i.e. the wording of this term is to be understood by the skilled person as being synonymous to "The process according to any one of embodiments 1, 2, 3 and 4". Further, it is explicitly noted that the following set of embodiments is not the set of claims determining the extent of protection, but represents a suitably structured part of the description directed to general and preferred aspects of the present invention.
1. A process for converting alcohols to aromatic hydrocarbons comprising,
   (1) preparing a feed comprising one or more alcohols having two or more carbon atoms;
   (2) providing a dehydration catalyst;
   (3) contacting the feed prepared in (1) with the dehydration catalyst provided in (2) and conducting a reaction, obtaining a mixture comprising one or more olefin and water;
   (4) optionally separating at least a portion of the water from the mixture obtained in (3) or optionally adding water to the mixture obtained in (3);
   (5) providing a catalyst comprising a zeolite;
   (6) contacting the mixture obtained in (3) or in (4) with the catalyst provided in (5), obtaining a mixture comprising one or more aromatic hydrocarbons as a reaction product;
   (7) separating at least a part of the aromatic hydrocarbons from the reaction product obtained in (6).
2. The process according to embodiment 1, wherein the one or more alcohols in (1) comprise, preferably consist of, one or more of ethanol, propanol, butanol, and isobutanol, preferably comprise, more preferably consist of, one or more of ethanol and propanol, more preferably comprise, more preferably consist of, ethanol.
3. The process according to embodiment 1 or 2, wherein the alcohol in (1) is the product of a fermentation process, preferably of the fermentation of biological material obtained from harvested plants and/or residual products from harvested plants.
4. The process according to any of embodiments 1 to 3, wherein the feed in (1) further comprises water.
5. The process according to embodiment 4, wherein the water content of the feed in (1) is 80 vol.-% or higher, preferably 85 vol.-%or higher, more preferably 90 vol.-% or higher, more preferably 95 vol.-% or higher.
6. The process according to any of embodiments 1 to 5, wherein the dehydration catalyst in (2) comprises, preferably consists of, one or more acid catalysts, preferably one or more solid acid catalysts and/or ionic liquids, wherein the one or more solid acid catalysts preferably comprise, more preferably consist of, one or more selected from the group consisting of alumina, sulfated zirconia, and zeolites, including mixtures of two or more thereof, more preferably comprise, more preferably consist of, one or more selected from the group consisting of gamma-alumina and MFI-type zeolites, including mixtures of two or more thereof, more preferably comprise, more preferably consist of, one or more selected from the group consisting of activated gamma-alumina and ZSM-5, including mixtures of two or more thereof.
7. The process according to any of embodiments 1 to 6, wherein the dehydration catalyst in (2) further comprises a binder, wherein the binder preferably comprises, more preferably consists of, one or more selected from the group consisting of titania, zirconia, alumina, silica, silica-alumina, titania-silica, titania-alumina, zirconia-silica, zirconia-alumina, titania-zirconia, and mixtures of two or more thereof, more preferably from the group consisting of silica-alumina, titania-silica, titania-alumina, zirconia-silica, zirconia-alumina, titania-zirconia, and mixtures of two or more thereof, wherein more preferably the binder comprises, more preferably consists of, silica-alumina.
8. The process according to any of embodiments 1 to 7, wherein the dehydration catalyst in (2) is provided as a shaped body, preferably as an extrudate.
9. The process according to any of embodiments 1 to 8, wherein from 95 to 100 wt.-% of the dehydration catalyst provided in (2) consists of the zeolite and the optional binder, preferably from 97 to 100 wt.-%, more preferably from 98 to 100 wt.-%, more preferably from 99 to 100 wt.-%, based on the total weight of the catalyst.
10. The process according to any of embodiments 1 to 9, wherein contacting in (3) is conducted at a temperature in the range of from 250 to 600 °C, preferably from 275 to 550 °C, more preferably 290 to 400 °C, more preferably from 300 to 450 °C, more preferably from 300 to 420 °C.
11. The process according to any of embodiments 1 to 10, wherein contacting in (3) is conducted at a pressure in the range of from 0.5 to 1.3 bara, preferably from 0.7 to 1.2 bara, more preferably from 0.9 to 1.1 bara.
12. The process according to any of embodiments 1 to 11, wherein the feed in (1) further comprises one or more inert gases, preferably nitrogen and/or argon, more preferably nitrogen.
13. The process according to any of embodiments 1 to 12, wherein the gas hourly space velocity at which the feed is contacted with the catalyst in (3) is in the range of from 600 to 2000 h⁻¹, preferably from 700 to 1600 h⁻¹, more preferably from 800 to 1300 h⁻¹, more preferably from 900 to 1050 h⁻¹.
14. The process according to any of embodiments 1 to 13, wherein the feed in (1) is a feed stream and contacting in (3) is conducted as a continuous process.
15. The process according to any of embodiments 1 to 13, wherein contacting in (3) is conducted as a batch process.
16. The process according to any of embodiments 1 to 15, wherein the mixture obtained in (3) comprises one or more of ethylene, propylene, and butene, preferably one or more of ethylene and propylene, more preferably ethylene.
17. The process according to any of embodiments 1 to 16, wherein the feed provided in (1) and contacted with the catalyst in (3) is in the liquid phase and/or in the gas phase, preferably in the gas phase.
18. The process according to any one of embodiments 1 to 17, further comprising (8) recycling the remaining reaction product comprising one or more unreacted olefins obtained in (7) to the feed prepared in (1) and/or to the mixture obtained in (3) and/or to the mixture contacted with the catalyst in (6), preferably to the mixture obtained in (3) and/or to the mixture contacted with the catalyst in (6), more preferably to the mixture contacted with the catalyst in (6).
19. The process according to any one of embodiments 1 to 17, further comprising
   (8) recycling one or more unreacted olefins obtained in (7) to the feed prepared in (1) and/or to the mixture obtained in (3) and/or to the mixture contacted with the catalyst in (6), preferably to the mixture obtained in (3) and/or to the mixture contacted with the catalyst in (6), more preferably to the mixture contacted with the catalyst in (6),
   wherein the one or more unreacted olefins comprise one or more olefins selected from the group consisting of C2 to C10 olefins, preferably C2 to C9 olefins, more preferably C2 to C8 olefins, more preferably C2 to C5 olefins, more preferably C2 to C4 olefins, more preferably C2 to C3 olefins, more preferably wherein the one or more unreacted olefins is ethylene.
20. The process according to any of embodiments 1 to 19, wherein the zeolite in (5) has an AEI, AFN, AFT, AFX, AFY, BSU, CGS, CHA, CON, DFT, EDI. ERI. ETV, IFY, IFW, IMF, ITH, ITR, IWR, JSR, JST, LOV, MFI, MER, MEL, OSO, PHI, POR, PUN, RSN, SAT, SBN, SFW, SIV, SOF, STW, SWY, SZR, THO, or UOS type framework structure, preferably an AEI, CGS, CHA, IFY, IMF, ITH, ITR, MFI, MER, POR, PUN, SZR, or UOS type framework structure, more preferably an MFI, MER, or SZR type framework structure, more preferably has an MFI type framework structure.
21. The process according to any of embodiments 1 to 20, wherein the zeolite in (5) has an MFI type framework structure, and wherein the zeolite is ZSM-5, Silicailite, Bor-C, LZ-105, AMS-1B, FZ-1, TZ-01, USC-4, NU-5, ZMQ-TB, TS-1, USI-108, AZ-1, TSZ, ZKQ-1B, NU-4, ZBH, or TSZ-III, preferably is ZSM-5.
22. The process according to any of embodiments 1 to 21, wherein the zeolite in (5) is a zeolite loaded with Ga, Zn, or Ni, or with combinations of two or more thereof, preferably with Zn or Ni, or with Zn and Ni, more preferably with Zn.
23. The process according to any of embodiments 1 to 22, wherein the zeolite in (5) is a zeolite loaded with Zn in the range of from 0.1 to 10 wt.-% calculated as the element and based on 100 wt.-% of the loaded zeolite, preferably from 0.2 to 8 wt.-%, more preferably from 0.3 to 7 wt.-%, more preferably from 0.5 to 5 wt.-%, more preferably from 0.7 to 3 wt.-%, more preferably from 0.9 to 1.5 wt.-%, more preferably from 1.0 to 1.3 wt.-%.
24. The process according to any of embodiments 1 to 23, wherein the zeolite in (5) is a zeolite loaded with Ga in the range of from 0.01 to 1 wt.-% calculated as the element and based on 100 wt.-% of the loaded zeolite, preferably from 0.05 to 0.6 wt.-%, more preferably from 0.075 to 0.5 wt.-%, more preferably from 0.1 to 0.4 wt.-%, more preferably from 0.15 to 0.3 wt.-%.
25. The process according to any of embodiments 1 to 23, wherein the zeolite in (5) is a zeolite loaded with Ga in the range of from 1 to 5 wt.-% calculated as the element and based on 100 wt.-% of the loaded zeolite, preferably from 1.5 to 2.5 wt.-%, more preferably from 1.7 to 2.3 wt.-%, more preferably from 1.8 to 2.2 wt.-%, more preferably from 1.9 to 2.1 wt.-%.
26. The process according to any of embodiments 1 to 25, wherein the zeolite in (5) is a zeolite loaded with Ni in the range of from 0.1 to 3 wt.-% calculated as the element and based on 100 wt.-% of the loaded zeolite, preferably from 0.5 to 1.5 wt.-%, more preferably from 0.7 to 1.3 wt.-%, more preferably from 0.8 to 1.2 wt.-%, more preferably from 0.9 to 1.1 wt.-%.
27. The process according to any of embodiments 1 to 26, wherein the zeolite in (5) is further loaded with P, Mg, or Ca, or with combinations of two or more thereof, preferably with P or Mg, or with P and Mg, more preferably with P.
28. The process according to any of embodiments 1 to 27, wherein the zeolite in (5) is further loaded with P in the range of from 0.1 to 5 wt.-% calculated as the element and based on 100 wt.-% of the loaded zeolite, preferably from 1 to 2 wt.-%, more preferably from 1.2 to 1.8 wt.-%, more preferably from 1.3 to 1.7 wt.-%, more preferably from 1.4 to 1.6 wt.-%.
29. The process according to any of embodiments 1 to 28, wherein the zeolite in (5) is further loaded with Mg in the range of from 0.1 to 5 wt.-% calculated as the element and based on 100 wt.-% of the loaded zeolite, preferably from 1 to 2 wt.-%, more preferably from 1.2 to 1.8 wt.-%, more preferably from 1.3 to 1.7 wt.-%, more preferably from 1.4 to 1.6 wt.-%.
30. The process according to any of embodiments 1 to 29, wherein the zeolite in (5) is further loaded with Ca in the range of from 0.1 to 5 wt.-% calculated as the element and based on 100 wt.-% of the loaded zeolite, preferably from 1 to 2 wt.-%, more preferably from 1.2 to 1.8 wt.-%, more preferably from 1.3 to 1.7 wt.-%, more preferably from 1.4 to 1.6 wt.-%.
31. The process according to any of embodiments 1 to 30, wherein the zeolite in (5) comprises YO₂ and X₂O₃ in its framework structure, wherein Y stands for a tetravalent element and X stands for a trivalent element.
32. The process according to embodiment 31, wherein X is selected from the group consisting of Al, B, Ga, and combinations of two or more thereof, wherein X is preferably Al.
33. The process according to embodiment 31 or 32, wherein Y is selected from the group consisting of Si, Ti, Sn, Ge, and combinations of two or more thereof, wherein Y is preferably Si.
34. The process according to any of embodiments 1 to 33, wherein the zeolite in (5) comprises Si and Al in its framework structure, wherein the Si:Al molar ratio of the zeolite in (2) ranges from 1 to 150, preferably from 3 to 120, more preferably from 5 to 110, more preferably from 30 to 100, more preferably from 40 to 80, more preferably from 50 to 70.
35. The process according to any of embodiments 1 to 34, wherein the surface area of the zeolite in (5) ranges from 250 to 550 m²/g, preferably from 275 to 525 m²/g, more preferably from 300 to 500 m²/g, more preferably from 325 to 475 m²/g, more preferably from 350 to 450 m²/g.
36. The process according to any of embodiments 1 to 35, wherein the catalyst in (5) further comprises a binder, wherein the binder preferably comprises, more preferably consists of, one or more selected from the group consisting of titania, zirconia, alumina, silica, silica-alumina, titania-silica, titania-alumina, zirconia-silica, zirconia-alumina, titania-zirconia, and mixtures of two or more thereof, more preferably from the group consisting of silica-alumina, titania-silica, titania-alumina, zirconia-silica, zirconia-alumina, titania-zirconia, and mixtures of two or more thereof, wherein more preferably the binder comprises, more preferably consists of, silica-alumina.
37. The process according to any of embodiments 1 to 36, wherein the catalyst in (5) is provided as a shaped body, preferably as an extrudate.
38. The process according to any of embodiments 1 to 37, wherein from 95 to 100 wt.-% of the catalyst provided in (5) consists of the zeolite and the optional binder, preferably from 97 to 100 wt.-%, more preferably from 98 to 100 wt.-%, more preferably from 99 to 100 wt.-%, based on the total weight of the catalyst.
39. The process according to any of embodiments 1 to 38, wherein the process includes a step of regenerating the catalyst after contacting with the feed in (6) wherein the catalyst is preferably regenerated by calcination at a temperature in the range of from 300 to 800 °C, preferably from 350 to 700 °C, more preferably from 400 to 600 °C, more preferably from 450 to 500 °C.
40. The process according to embodiment 39, wherein the calcination is conducted for a period ranging from 1 to 24 h, preferably from 2 to 12 h, more preferably from 3 to 8 h.
41. The process according to any of embodiments 1 to 40, wherein contacting in (3) or (6), preferably in (3) and (6), is conducted in a reactor, preferably in a fixed bed reactor or in a fluidized bed reactor, more preferably in a fixed bed reactor.
42. The process according to any of embodiments 1 to 41, wherein contacting in (6) is conducted at a temperature in the range of from 350 to 600 °C, preferably from 370 to 550 °C, more preferably 390 to 530 °C, more preferably from 410 to 500 °C, more preferably from 420 to 480 °C.
43. The process according to any of embodiments 1 to 42, wherein contacting in (6) is conducted at a pressure in the range of from 0.5 to 1.3 bara, preferably from 0.7 to 1.2 bara, more preferably from 0.9 to 1.1 bara.
44. The process according to any of embodiments 1 to 43, wherein the gas hourly space velocity at which the mixture obtained in (3) or (4) is contacted with the catalyst in (5) is in the range of from 1500 to 2500 h⁻¹, preferably from 1600 to 2200 h⁻¹, more preferably from 1700 to 2150 h⁻¹, more preferably from 1800 to 2100 h⁻¹, more preferably from 1900 to 2050 h⁻¹.
45. The process according to any of embodiments 1 to 44, wherein the mixture obtained in (3) or (4) is a feed stream and contacting in (6) is conducted as a continuous process, wherein preferably the process is conducted as a continuous process.
46. The process according to any of embodiments 1 to 44, wherein contacting in (6) is conducted as a batch process, wherein preferably the process is conducted as a batch process.
47. The process according to any of embodiments 1 to 46, wherein the aromatic hydrocarbons obtained in (6) comprise one or more of benzene, toluene, and xylene, preferably one or more of benzene and toluene, more preferably benzene.
48. The process according to any of embodiments 1 to 47, wherein the mixture obtained in (3) or (4) and contacted with the catalyst in (6) is in the liquid phase and/or in the gas phase, preferably in the gas phase.
49. The process according to any of embodiments 1 to 48, wherein the total yield of benzene, toluene, and xylene (BTX) obtained in (6) is 35 % or higher based on 100% of the one or more olefins which are converted in (6), preferably 40 % or higher, more preferably 45 % or higher, more preferably 50 % or higher, more preferably 55 % or higher.
50. The process according to any of embodiments 1 to 49, wherein (6) comprises at least one reaction stage (A), wherein in (A)
   (6.1) contacting is carried out in at least two reactors RA1, ... , RAn connected in parallel, wherein each reactor contains the catalyst provided in (5);
   (6.2) at a given point in time during contacting, at least one of the at least two reactors RA1, ... , RAn is out of operation for regenerating the catalyst present, so that at least 1 reactor remains in operation.
51. The process according to embodiment 50, wherein (6) further comprises (6.3) uniting the mixtures comprising one or more aromatic hydrocarbons as a reaction product obtained according to (6.1) of the at least two reactors RA1, ... , RAn.
52. The process according to any of embodiments 1 to 51, wherein the process is operated without using combustion as energy source, wherein the process is preferably operated using only electric energy as energy source, more preferably using only renewable electric energy as energy source, more preferably using only energy from renewable energy sources as energy source.
53. The process according to any one of embodiments 1 to 52, wherein independently from each other separating in (7) is conducted by distillation, preferably by fractional distillation.

### EXPERIMENTAL SECTION

The following part aims at a more detailed description of the process for the preparation of catalyst materials, the properties of said catalyst materials, and the process for the conversion of alcohol containing feeds by using said catalyst materials according to the invention. It is noted that the examples do not limit the scope of the inventions but represent the diversity of the different aspects of the invention under specially selected process conditions.

### Reference Example 1: Determination of surface area

The surface area determination was carried out via nitrogen adsorption at 77 K according to DIN 66131.

### Reference Example 2: Determination of reaction products

The reaction products were determined by a gas chromatograph from HP (Agilent 7890 A) with a 50 m column and FID analyzer (FID1: RT-Q BOND 30 m, FID 2: customized 30 m HTE column, TCD: RT-Q BOND 30 m and molsieve column)

### Reference Example 3: Preparation of the zeolite catalyst

### Preparation of the zeolite

In a 2 m³ reactor 79.61 kg of distilled water is first introduced. To the water, 411.15 kg of an aqueous tetrapropylammonium hydroxide solution (TPAOH; 40 wt.-%) was added under stirring (70 rpm). The suspension is let for stirring for another 10 min. 8.2 kg solid NaOH is added slowly in 2.5 kg portions under stirring and after each portion the system is allowed to mix for 5 minutes. Next, 29.25 kg aluminium triisopropoxide is added to the suspension and the system is stirred for another 1 h. At the end, 538.19 kg colloidal silica (Ludox AS-40) is added followed by additional 10 kg of distilled water. The synthesis mixture is stirred another 1 h at room temperature before the reactor is flushed with nitrogen gas and the pressure reduced to -900 mbar. Afterwards the reactor is heated to 170 °C in 11 h. The hydrothermal synthesis is run for 72 h at 170 °C under 70 rpm stirring. After crystallization the synthesis mixture is cooled down to 30 °C. The suspension is transferred to a larger vessel where the pH of the suspension is adjusted to 7 ± 0.5, by addition of a 10 wt.-% aqueous nitric acid solution. The pH adjusted suspension is let for stirring for another 30 min at 70 rpm. The zeolite is separated by filtration and the filter cake is washed with distilled water until a conductivity of the wash water < 200 µS. The filter cake is then dried at 120 °C for 96 h. The dried material was calcined to 550 °C in air for 6 h for obtaining a calcined ZSM-5 zeolite with a BET surface area of 390 m²/g, and displaying a crystallinity as determined by X-ray diffraction of 94 %.

250 kg distilled water is added to a 400 L reactor and 25 kg ammonium nitrate is added under stirring (150 rpm). The suspension is heated to 80 °C, followed by the addition of 25 kg of the calcined zeolite. The mixture is stirred further for 1 h at 80 °C. Afterwards the reaction mixture is cooled down and filtered off using a filter press and washed with water until a conductivity in the wash water < 200 µS. The ion-exchange process is then repeated for obtaining an ammonium exchanged ZSM-5. The filter cake obtained after the second ammonium ion-exchange process is dried for 10 h at 120 °C and calcined at 500 °C in air for 5 h (heating rate 2 °C/ min) for obtaining ZSM-5 in the H-form.

### Preparation of the zeolite loaded with Zn

The preparation of the catalyst materials was based on a procedure by incipient wetness impregnation. The zeolite is contacted with an aqueous solution of a Zn- salt. The impregnated sample was dried at 100 °C for 5 hours in a laboratory oven under air. The drying step was then followed by a thermal treatment at 500 °C for 3 hours under a reducing atmosphere (which was provided by H₂/He = 50/50). The resulting catalyst displayed a Zn loading of 1.1 wt.-%, calculated as the respective element.

### Preparation of the zeolite catalyst loaded with Zn and P

The preparation of the catalyst materials was based on a procedure by incipient wetness impregnation. The zeolite was contacted with an aqueous solution of a Zn- salt. The impregnated sample was dried at 120 °C for 16 hours in a laboratory oven under air. The drying step was then followed by a thermal treatment at 500 °C for 5 hours (heating rate of 2 °C/min), also under air. In a subsequent step, the material was impregnated with P, by incipient wetness impregnation using an 85 % H₃PO₄ solution. The impregnated sample was dried at 120 °C for 4 hours in a laboratory oven under air. The drying step was then followed by a thermal treatment at 500 °C for 5 hours (heating rate of 2 °C/min), also under air. The resulting catalyst displayed a Zn loading of 1.1 wt.-% and a P loading of 1.3 wt.-%, calculated as the respective element and was sieved to powder (250-315µm).

### Preparation of the zeolite loaded with Zn and Ca

The preparation of the catalyst material was performed analogue to the preparation of the zeolite catalyst loaded with Zn and P, but instead of a H₃PO₄ solution a Ca(NO₃)₂ solution was used to impregnate the sample with Ca. The resulting catalyst displayed a Zn loading of 1.2 wt.-% and a Ca loading of 1.2 wt.-% calculated as the respective element.

### Preparation of the zeolite loaded with Zn and Ga

The preparation of the catalyst material was performed analogue to the preparation of the zeolite catalyst loaded with Zn and P, but instead of a H₃PO₄ solution a Ga(NO₃)₃ solution was used to impregnate the sample with Ga. The resulting catalyst displayed a Zn loading of 8.5 wt.-% and a Ga loading of 1.6 wt.-% calculated as the respective element.

**Table 1: Summary of the tested zeolite catalyst materials.**

| Experiment | Loaded element | [wt.-%] |
|---|---|---|
| Example 3.1 | Zn | Zn: 1.1 |
| Example 3.2 | Zn & P | Zn: 1.1, P: 1.3 |
| Example 3.3 | Zn & Ca | Zn: 1.2, Ca: 1.2 |
| Example 3.4 | Zn & Ga | Zn: 8.5, Ga: 1.6 |
| Example 3.5 | Zn | Zn: 4.2 |

### Reference Example 4: Testing of different feed compositions

Catalytic testing experiments were conducted in a high throughput testing apparatus which could be equipped with a set of sixteen tubular reactors. The tubular reactors were made of stainless steel (1.4571) and had a length of 0.51 m and an inner diameter of 6 mm. For the preparation of the catalytic test experiments the reactors were filled with the catalyst (as split fraction by using the particles in the range from 250 to 315 µm). Each reactor was filled with 1 ml of catalyst sample which was placed on top of inert particles (silicon carbide, F60 was used as inert material). A volume of 1 ml of catalyst corresponds to a weight in the range from 0.5 to 0.9 g catalyst material. The catalyst was over layered by inert particles. The quantity and filling level were registered. The filled reactors were inserted in the testing apparatus, which could be used in a large operating field of different testing parameters. The high throughput testing apparatus was equipped with online-analytical equipment which was connected to the exit tubes of the of the outlets of the reactor tubes in such a way that the product streams could be subjected to online characterization by the analytical equipment. The operation of the high throughput testing apparatus and the analytical testing equipment was controlled by computer program that had been developed for the kind of setup and which is characterized by high accuracy.

Three different feeds were used. TC2 (EtOH/Ar, 90/10) represents the feed in a one step preparation process starting from ethanol. TC3 (Ethene/H2O/Ar 45/45/10) represents the feed, wherein prior to a second stage a feed comprising ethanol was treated with an upstream first dehydration stage and converted to ethene. TC4 (Ethene/Ar 90/10) represents the feed, wherein prior to the second stage a feed comprising ethanol was treated with an upstream first dehydration stage and converted to ethane and wherein prior to the second stage water was separated from the product feed of stage 1.

One of the criteria according to which the process properties were examined was given by the BTX-selectivities. BTX-selectivities are given by the relative yield of the components benzene, toluene, and xylene. For the group of benzene, toluene, and xylene the abbreviation BTX is used. The catalytic test experiments were carried out in a time range of from two to sixty hours. During the catalytic test experiments, online-analyses were carried out on the product fluid stream by GC measurements. By these measurements changes could be detected as a result of decreasing catalyst activity or change in select ivies. The duration of the catalytic test was depending on the performance characteristics of the individual catalyst materials. At the point in time when the activities or the selectivities had decreased significantly, the catalytic test experiments were stopped.

**Table 2: List of the selected test conditions with different feeds.**

| Experiments | Feed Nr. | Feed composition | Catalyst | GHSV [h⁻¹] | Temp. [°C] |
|---|---|---|---|---|---|
| Comparative Example 4.1 | TC2 | EtOH/Ar 90/10 | Example 3.2 | 1000 | 450 |
| Example 4.2 | TC3 | Ethene/H2O/Ar 45/45/10 | Example 3.2 | 2000 | 450 |
| Example 4.3 | TC4 | Ethene/Ar 90/10 | Example 3.2 | 1000 | 450 |
| Comparative Example 4.4 | TC2 | EtOH/Ar 90/10 | Example 3.3 | 1000 | 450 |
| Example 4.5 | TC3 | Ethene/H2O/Ar 45/45/10 | Example 3.3 | 2000 | 450 |
| Example 4.6 | TC4 | Ethene/Ar 90/10 | Example 3.3 | 1000 | 450 |
| Comparative Example 4.7 | TC2 | EtOH/Ar 90/10 | Example 3.4 | 1000 | 450 |
| Example 4.8 | TC3 | Ethene/H2O/Ar 45/45/10 | Example 3.4 | 2000 | 450 |
| Example 4.9 | TC4 | Ethene/Ar 90/10 | Example 3.4 | 1000 | 450 |
| Comparative Example 4.10 | TC2 | EtOH/Ar 90/10 | Example 3.5 | 1000 | 450 |
| Example 4.11 | TC3 | Ethene/H2O/Ar 45/45/10 | Example 3.5 | 2000 | 450 |
| Example 4.12 | TC4 | Ethene/Ar 90/10 | Example 3.5 | 1000 | 450 |

As may be taken from Table 2, comparative example 4.1, 4.4, 4.7 and 4.10 represents a feed in a process in which ethanol is directly fed into the reactor for conversion thereof to benzene, toluene, and xylene (BTX). Inventive example 4.2, 4.5, 4.8 and 4.11 represents a feed in a process in which ethanol is fed into a first reactor located upstream of the reactor for conversion to BTX, wherein ethanol is dehydrated in said first reactor, thus affording a feed containing equimolar amounts of water and ethylene. Inventive example 4.3, 4.6, 4.9 and 4.12 on the other hand, represents a feed in a process, wherein water has been separated from the product feed of the upstream first reactor prior to the feed being fed into the reactor for conversion thereof to BTX, thus affording a feed containing ethylene.

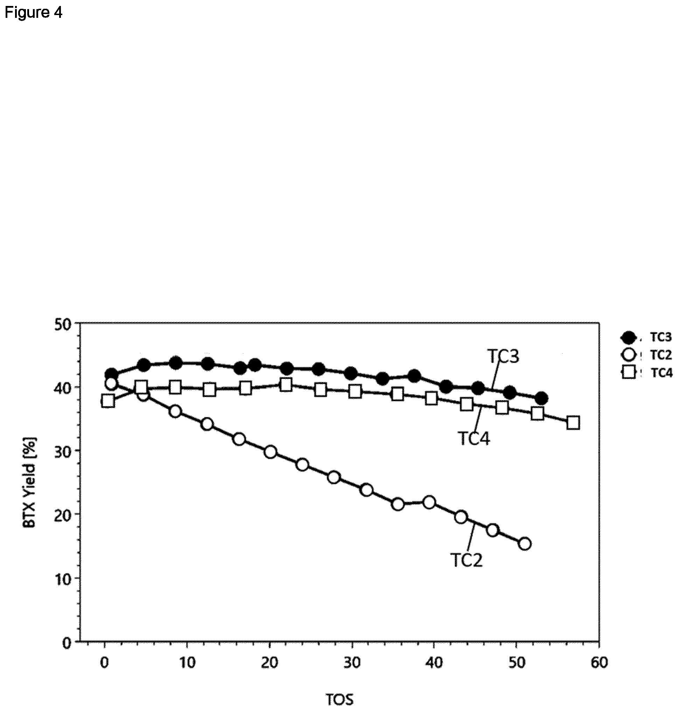

The results from testing are displayed in Figures 4 to 7. Thus as may be taken from the results, it has quite unexpectedly been found that the process according to the invention employing a first stage of dehydration of ethanol and feeding the resulting mixture of water and ethylene into the process stage for conversion to BTX affords not only the highest yields compared to the one stage processes departing from either ethanol or ethylene, but furthermore also affords said improved result during the entire time on stream up to the regeneration of the catalyst.

A further surprising result was that the catalyst materials could be regenerated in such a way that the performance properties could be completely restored as with the original state. It was also particularly surprising that the catalyst regeneration could be efficiently performed over a large number of cycles by switching between the conditions of process operation and regeneration.

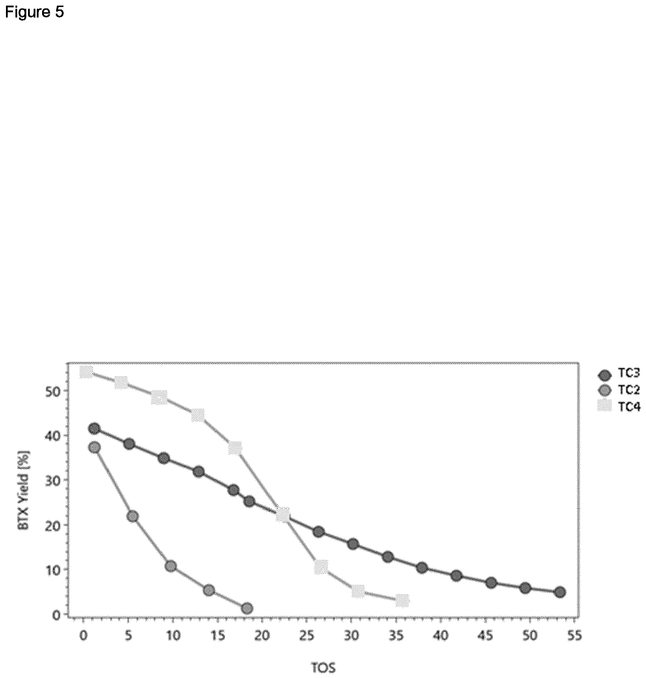

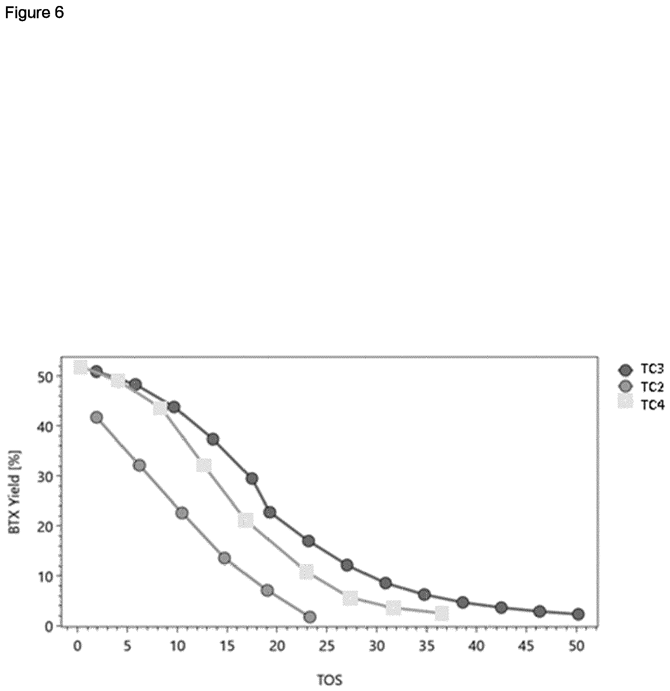

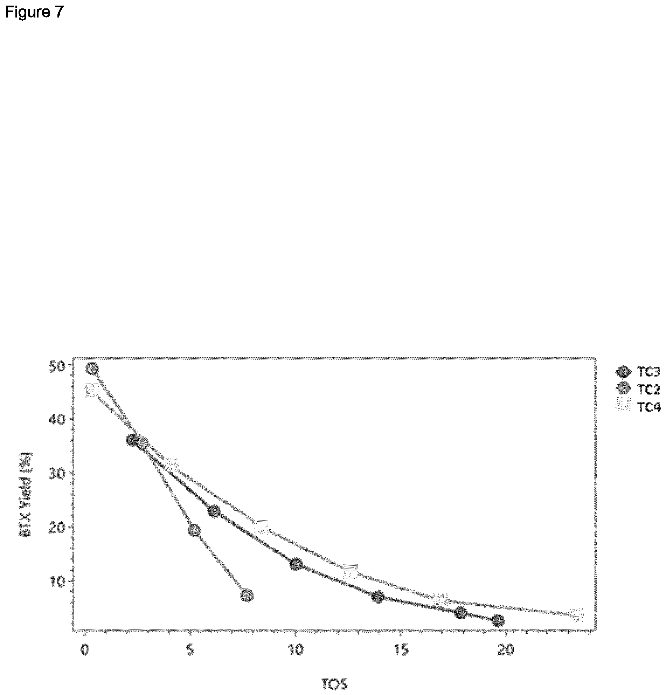

Finally, inventive example 4.3, 4.6, 4.9 and 4.12 represent the inventive process in which water in the product stream from the first stage is entirely separated for affording an essentially pure ethylene feed stream for the second process stage. Although, as may be taken from Figures 5 and 7 the second process stage shows a slightly lower conversion level to BTX than when employing a stream containing ethylene and water, the process of inventive examples 4.3, 4.6, 4.9 and 4.12 has the advantage that water must not be removed in a subsequent stage, wherein its removal may be more energy- and cost-intensive than when performed between the first and second process stages. Further, inventive examples 4.3 and 4.9 surprisingly show an increased conversion of the feed containing water (TC3) compared to the feed without water (TC4) (see figures 4 and 6). Furthermore, the inventive process would have the advantage that crude ethanol containing water may be employed for the first process stage, wherein a separation of water from the resulting ethylene containing product stream is highly energy- and cost-efficient compared to the separation of water from the crude ethanol stream.

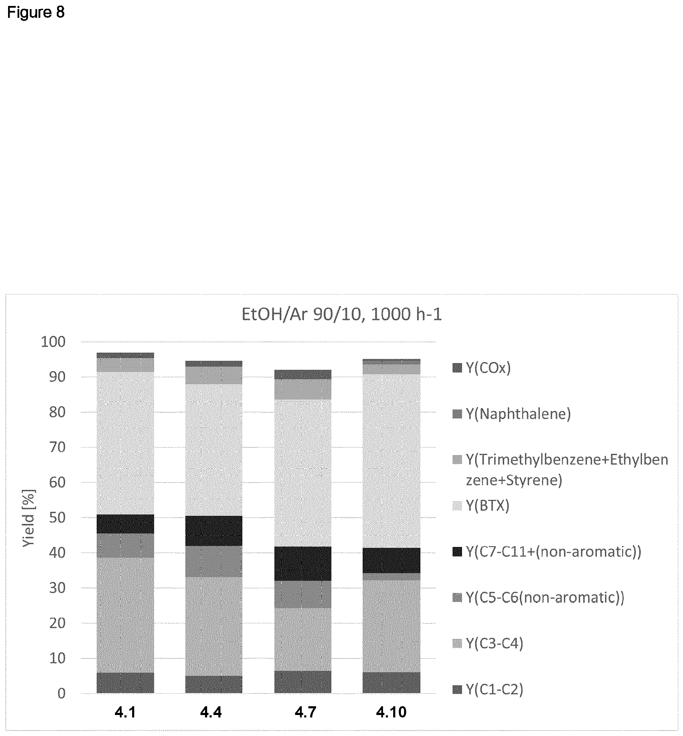

The product distribution of all tested samples is shown in Figures 8 to 10.

**Table 3: List of compounds and their abbreviation in Figures 8 to 10.**

| Abbreviation | Compound |
|---|---|
| C1P | Methane |
| C2P | Ethane |
| C3P | Propane |
| iC4P | Isobutane |
| nC4P | n-Butane |
| iC5P | Isopentane |
| nC5P | n-pentane |
| C6-C11 | Hexanes to undecanes |
| C2O | Ethylene |
| C3O | Propylene |
| iC4O | Isobutylene |
| nC4O | n-butylene |
| iC5O | isopentylene |
| C6-C9O | C6-C9 olefines |
| C6A | Benzene |
| C7A | Toluene |

### DESCRIPTION OF THE FIGURES

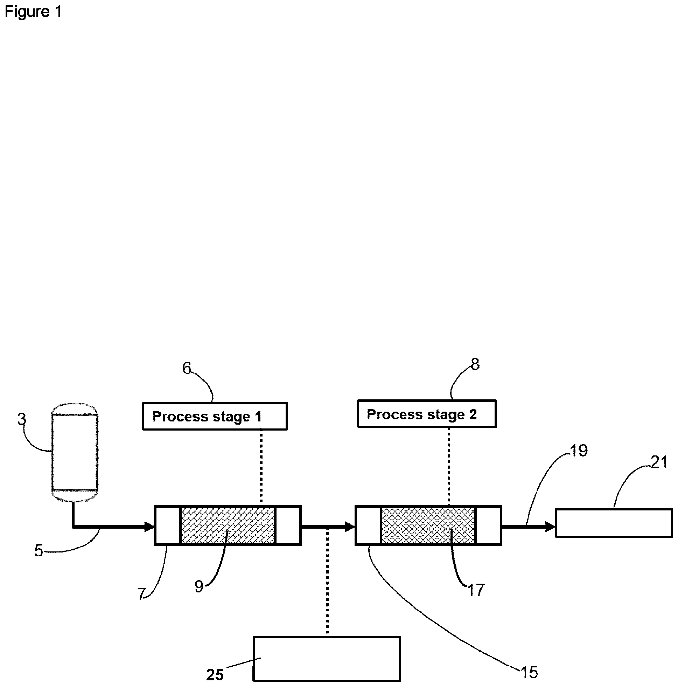

- Figure 1: is a schematic representation of a production unit used for the process according to an embodiment of the invention, wherein ethanol is first fed to a dehydration reactor (Process stage 1), and the product stream from said stage is then fed to a reactor for the conversion to aromatic hydrocarbons (Process stage 2).

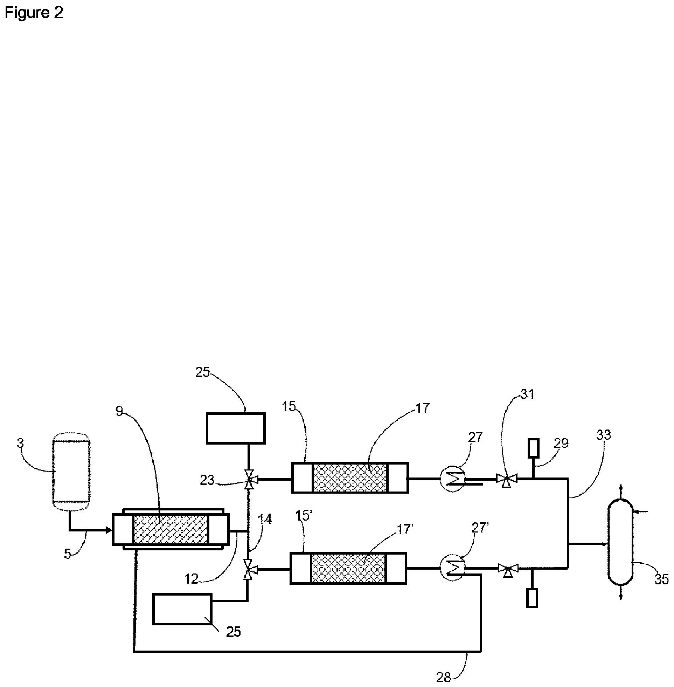

- Figure 2: is a schematic representation of a production unit used for the process according to an embodiment of the invention, wherein the reaction of the product stream from the first process stage (dehydration reactor) is fed to two reactors connected in parallel in the subsequent stage of the conversion to aromatic hydrocarbons.

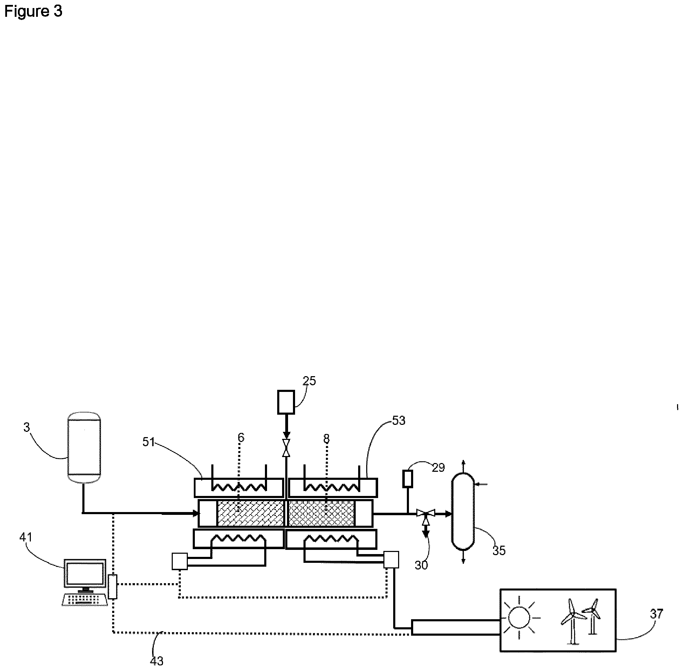

- Figure 3: is a schematic representation of a production unit used for the process according to an embodiment of the invention, wherein the reactors are respectively heated with electricity obtained from sustainable sources of energy.
- Figure 4: shows the results from testing conducted according to reference example 4 with the catalyst of Example 3.2 prepared according to Reference Example 3. In the diagram, the BTX yield in % is plotted along the ordinate and the time on stream (TOS) is plotted along the abscissa. In the diagram, TC2 represents the results for the ethanol feed employed in comparative example 4.1, TC3 represents the results for the feed wherein ethanol was dehydrated prior to the reaction in accordance with example 4.2, and TC4 represents the results for the ethylene feed employed in example 4.3.
- Figure 5: shows the results from testing conducted according to reference example 4 with the catalyst of Example 3.3 prepared according to Reference Example 3. In the diagram, the BTX yield in % is plotted along the ordinate and the time on stream (TOS) is plotted along the abscissa. In the diagram, TC2 represents the results for the ethanol feed employed in comparative example 4.4, TC3 represents the results for the feed wherein ethanol was dehydrated prior to the reaction according to example 4.5, and TC4 represents the results for the ethylene feed employed in example 4.6.
- Figure 6: shows the results from testing conducted according to reference example 4 with the catalyst of Example 3.4 prepared according to Reference Example 3. In the diagram, the BTX yield in % is plotted along the ordinate and the time on stream (TOS) is plotted along the abscissa. In the diagram, TC2 represents the results for the ethanol feed employed in comparative example 4.7, TC3 represents the results for the feed wherein ethanol was dehydrated prior to the reaction according to example 4.8, and TC4 represents the results for the ethylene feed employed in example 4.9.
- Figure 7: shows the results from testing conducted according to reference example 4 with the catalyst of Example 3.5 prepared according to Reference Example 3. In the diagram, the BTX yield in % is plotted along the ordinate and the time on stream (TOS) is plotted along the abscissa. In the diagram, TC2 represents the results for the ethanol feed employed in comparative example 4.10, TC3 represents the results for the feed wherein ethanol was dehydrated prior to the reaction according to example 4.11, and TC4 represents the results for the ethylene feed employed in example 4.12.
- Figure 8: shows the product distribution of Comparative Examples 4.1, 4.4, 4.7 and 4.10. The product distribution legend is listed in table 3. In the diagram, the yield in % is plotted along the ordinate.

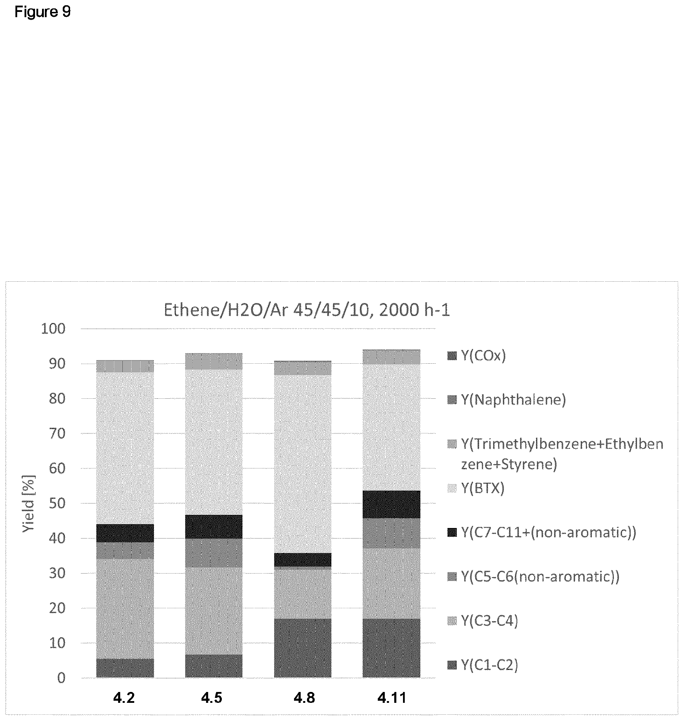

- Figure 9: shows the product distribution of Inventive Examples 4.2, 4.5, 4.8 and 4.11. The product distribution legend is listed in table 3. In the diagram, the yield in % is plotted along the ordinate.

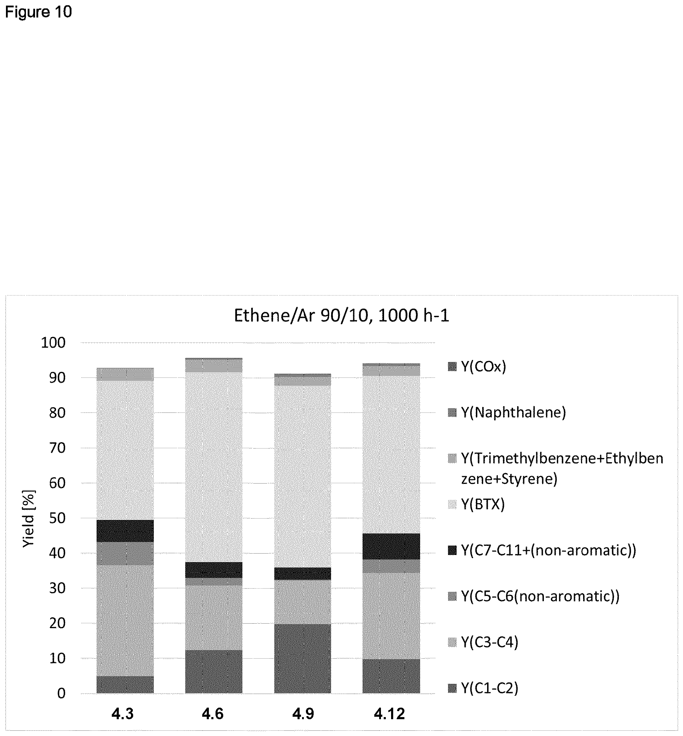

- Figure 10: shows the product distribution of Inventive Examples 4.3, 4.6, 4.9 and 4.12. The product distribution legend is listed in table 3. In the diagram, the yield in % is plotted along the ordinate.

**REFERENCE NUMBERS**

| | |
|---|---|
| 3 - | feed tank |
| 5 - | supply line |
| 6 - | first process stage |
| 7 - | reactor |
| 8 - | second process stage |
| 9 - | catalyst |
| 11 - | output line |
| 12 - | output line of the first reactor stage |
| 13 - | collecting device |
| 14 - | distribution line |
| 15 - | reactor of the second stage |
| 17 - | catalyst in the reactors of the second stage |
| 15' - | reactor of the second stage in a parallel arrangement |
| 17' - | catalyst in the reactors of the second stage in parallel arrangement |
| 19 - | output line of the second reactor stage |
| 21 - | reaction product stream |
| 23, 30, 31 - | switching valve |
| 25 - | blank box can be (i) the introduction of a second feed for recycling unreacted olefin obtained in (7), (ii) the separation of water from the mixture obtained in (3), or (iii)the addition of water to the mixture obtained in (3) |
| 27, 27' - | heat exchanger |
| 28 - | heat recirculation |
| 29 - | sensor, analytical device |
| 33 - | transfer line |
| 35 - | distillation tower |
| 37 - | electric power grid |
| 41 - | process control / smart process control |
| 43 - | process control circuit |
| 51 - | heating unit of the process stage 6 |
| 53 - | heating unit of the process stage 8 |

### Cited prior art documents:

- Uslamin et al., Chemical Engineering Science 2019, 198, 305-316
- Zhang et al., Science 2020, 370, 437-441
- Uslamin et al., ACS Catal. 2019, 9, 8547-8554
- US 9,533,921 B2
- Uslamin et al., Catal. Sci. Technol. 2020, 10, 2774

## Claims

1. A process for converting alcohols to aromatic hydrocarbons comprising,
(1) preparing a feed comprising one or more alcohols having two or more carbon atoms;
(2) providing a dehydration catalyst;
(3) contacting the feed prepared in (1) with the dehydration catalyst provided in (2) and conducting a reaction, obtaining a mixture comprising one or more olefin and water;
(4) optionally separating at least a portion of the water from the mixture obtained in (3) or optionally adding water to the mixture obtained in (3);
(5) providing a catalyst comprising a zeolite;
(6) contacting the mixture obtained in (3) or in (4) with the catalyst provided in (5), obtaining a mixture comprising one or more aromatic hydrocarbons as a reaction product;
(7) separating at least a part of the aromatic hydrocarbons from the reaction product obtained in (6).

2. The process according to claim 1, wherein the one or more alcohols in (1) comprise, one or more of ethanol, propanol, butanol, and isobutanol.

3. The process according to claim 1 or 2, wherein the alcohol in (1) is the product of a fermentation process.

4. The process according to any of claims 1 to 3, wherein the feed in (1) further comprises water.

5. The process according to any one of claims 1 to 4, further comprising
(8) recycling the remaining reaction product comprising one or more unreacted olefins obtained in (7) to the feed prepared in (1) and/or to the mixture obtained in (3) and/or to the mixture contacted with the catalyst in (6).

6. The process according to any one of claims 1 to 5, further comprising
(8) recycling one or more unreacted olefins obtained in (7) to the feed prepared in (1) and/or to the mixture obtained in (3) and/or to the mixture contacted with the catalyst in (6),
wherein the one or more unreacted olefins comprise one or more olefins selected from the group consisting of C2 to C10 olefins.

7. The process according to any of claims 1 to 6, wherein the zeolite in (5) has an MFI type framework structure, and wherein the zeolite is ZSM-5, Silicailite, Bor-C, LZ-105, AMS-1B, FZ-1, TZ-01, USC-4, NU-5, ZMQ-TB, TS-1, USI-108, AZ-1, TSZ, ZKQ-1B, NU-4, ZBH, or TSZ-III.

8. The process according to any of claims 1 to 7, wherein the zeolite in (5) is a zeolite loaded with Ga, Zn, or Ni, or with combinations of two or more thereof.

9. The process according to any of claims 1 to 8, wherein the zeolite in (5) is a zeolite loaded with Zn in the range of from 0.1 to 10 wt.-% calculated as the element and based on 100 wt.-% of the loaded zeolite.

10. The process according to any of claims 1 to 9, wherein the zeolite in (5) is further loaded with P, Mg, or Ca, or with combinations of two or more thereof.

11. The process according to any of claims 1 to 10, wherein the zeolite in (5) is further loaded with P in the range of from 0.1 to 5 wt.-% calculated as the element and based on 100 wt.-% of the loaded zeolite.

12. The process according to any of claims 1 to 11, wherein the aromatic hydrocarbons obtained in (6) comprise one or more of benzene, toluene, and xylene.

13. The process according to any of claims 1 to 12, wherein (6) comprises at least one reaction stage (A), wherein in (A)
(6.1) contacting is carried out in at least two reactors RA1, ... , RAn connected in parallel, wherein each reactor contains the catalyst provided in (5);
(6.2) at a given point in time during contacting, at least one of the at least two reactors RA1, ... , RAn is out of operation for regenerating the catalyst present, so that at least 1 reactor remains in operation.

14. The process according to claim 13, wherein (6) further comprises (6.3) uniting the mixtures comprising one or more aromatic hydrocarbons as a reaction product obtained according to (6.1) of the at least two reactors RA1, ... , RAn.

15. The process according to any of claims 1 to 14, wherein the process is operated without using combustion as energy source.
